# EUROPEAN PATENT APPLICATION

(11) **EP 4 438 033 A1**
(43) Date of publication of application: **02.10.2024**
(21) Application number: 22899132.9
(22) Date of filing: 28.11.2022
(51) Int. Cl.: A61K 8/9794, A61K 8/11, A61Q 19/00, A61Q 19/10

(54) **COSMETIC COMPOSITION USING BREWER'S SPENT GRAIN AND METHOD FOR MANUFACTURING SAME**

(30) Priority: 26.11.2021 KR 20210165220; 26.11.2021 KR 20210165221; 26.11.2021 KR 20210165222
(71) Applicant: Rafiq Cosmetics Co. Ltd., Cheonan-si, Chungcheongnam-do 31094 (KR)
(72) Inventor: LEE, Pom Joo, Yongin-si Gyeonggi-do 16807 (KR); LEE, Ji Hoon, Seongnam-si Gyeonggi-do 13556 (KR)
(74) Representative: Viering, Jentschura & Partner mbB Patent- und Rechtsanwälte
(86) International application number: PCT/KR2022/019001
(87) International publication number: WO 2023/096450

(57) **Abstract**

A method, according to an embodiment of the present disclosure, includes: performing a sterilization process to dry a brewer's spent grain raw material and then performing a milling process on the resulting brewer's spent grain raw material; pre-formulating a brewer's spent grain scrub or capsule by classifying the milled brewer's spent grain raw material by a particle size condition and performing a post-process depending on a post-processing method; preparing an aqueous phase and an oil phase of a cosmetic ingredient for preparing a cosmetic containing a cosmetic composition containing the brewer's spent grain scrub or capsule by separately weighing each of the aqueous and oil phases; performing a heating process on each of the aqueous and oil phases through double boiling and then performing an emulsification process by stirring the aqueous phase and introducing the oil phase; performing a cooling process on the resulting composition obtained through the emulsification process; performing stirring by introducing the pre-formulated brewer's spent grain scrub or capsule; and performing a vacuum degassing process on the resulting stirred composition.

## Description

### Technical Field

The present disclosure relates to a cosmetic composition and a preparation method thereof. More specifically, the present disclosure relates to a cosmetic composition using a processed brewer's spent grain product and a preparation method thereof.

### Background Art

Various intrinsic and extrinsic factors, such as ultraviolet rays and stress, cause all types of skin breakouts in modern people and accelerate skin aging including melasma, freckles, and skin pigmentation (1. Voegeli, R. 1996. Elastase and typtase determination on human skin surface. Cosmetic &Toiletries. 111, 51-58.). In skin pigmentation, melanin pigments are known to control initial reactions from tyrosinase enzymes and L-tyrosine, such as DHICA oxidase (TRP-1), to 3,4-dihydroxyphenylalanine (DOPA) and from DOPA to DOPA quinone in biosynthesis (2. Aroca, P., et al. 1993. Melanin biosynthesis patterns of following hormonal stimulation. J. Biol Chem 268, 25650-25655.). On this basis, research is being actively in progress on exploring natural products that inhibit the activity of tyrosinase enzymes and thus can affect the inhibition of melanin biosynthesis. As a result, research using natural products, such as the inhibitory effect of tyrosinase gene expression using *Houttuynia cordata* extract (3. Chin, J. E., et al. 2005. Effects of Houttuynia cordata extracts on tyrosinse gene expression. J. Korean Soc Food Sci Nutr 34, 1284-1288.), has been actively conducted. Additionally, representative materials of currently known antioxidant and skin-whitening ingredients include arbutin, kojic acid, and ascorbic acid, and plant extracts from mulberry root bark, *Broussonetia kazinoki,* and licorice are widely known.

Brewer's spent grain (BSG) makes up approximately 80% to 90% of by-products generated during beer brewing processes. Wort is obtained by mashing beer barley, the main raw material in beer brewing, through germination and then adding malt to barley grains, a by-product obtained by separating the resulting mash. Brewer's spent grain is obtainable as residues of the obtained wort left after being processed to produce beer. Brewer's spent grain is composed primarily of the husk, pericarp, and seed coat layers of beer barley, has abundant minerals and vitamins, and is especially high in dietary fiber and protein content.

Brewer's spent grain contains large amounts of dietary fiber, essential amino acids, and the like, meaning that the nutritional value thereof is high considering the low price, and thus is highly useful as food and livestock feed. There have been actual reports that milk production increased in livestock fed with feed containing brewer's spent grain, and the weight gain of fish fed with feed containing brewer's spent grain was greater than that of fish fed only with rice bran. Brewer's spent grain contains large amounts of lactogenic growth hormones, such as prolactin hormones, and thus has long been used in sheep and cattle for growth, weight gain, and high-quality milk production. Additionally, regarding research on edibility, the commercial value of bread, flakes, biscuits, and the like using brewer's spent grain in the baking industry is being studied. When adding brewer's spent grain for actual use thereof in food, bread and cookies tasted better than before. As a result of clinical trials, effects, including reducing lipids and cholesterol in the body and reducing constipation, were exhibited. As described above, there is a good chance that brewer's spent grain, a food raw material providing high fiber, can be extensively applied to other industries in addition to food.

However, the brewer's spent grain, currently only used as livestock feed or food raw material, is discarded when failing to be used for this purpose.

### Disclosure

### Technical Problem

The present disclosure, which has been proposed to solve the problems described above, aims to provide a cosmetic composition using a processed brewer's spent grain product and a preparation method thereof, the cosmetic composition enabling brewer's spent grain to be used as a whole in an economically and environmentally friendly manner through processing and upcycling to be suitable for cosmetic ingredients based on the abundant fiber and active ingredients of brewer's spent grain.

### Technical Solution

A method, according to an embodiment for achieving the objectives of the present disclosure described above,
includes the following steps: performing a sterilization process on a brewer's spent grain raw material; performing a drying process on the sterilized brewer's spent grain raw material; performing a milling process on the resulting brewer's spent grain raw material obtained through the drying process; classifying the milled brewer's spent grain raw material by a particle size condition; determining a post-processing method corresponding to the classified particle size condition; and preparing a processed brewer's spent grain product for preparing a cosmetic composition by category, depending on the determined post-processing method.

Additionally, the particle size condition may correspond to a milling size condition, and the milling size condition may include at least one among the following conditions: less than 50 mesh, 50 mesh, 1000 mesh, and 3000 mesh.

Additionally, when the milling size condition corresponds to a fine powder, the post-processing method includes: a fine milling process performed on the milled brewer's spent grain raw material; a granulation process performed on a mixture of the resulting fine powder of the brewer's spent grain raw material and a capsule seed; and an encapsulation process performed on the resulting product through coating.

Additionally, when the milling size condition corresponds to 3000 mesh, the category of the cosmetic composition is characterized by corresponding to a body scrub, when the milling size condition corresponds to 1000 mesh, the category of the cosmetic composition is characterized by corresponding to a body cleanser, and when the milling size condition corresponds to 50 mesh, the category of the cosmetic composition is characterized by corresponding to a facial scrub.

Additionally, the post-processing method includes a process of obtaining a brewer's spent grain extract from the milled brewer's spent grain raw material by performing hot water extraction and filtration.

The cosmetic composition is formulated into a cosmetic containing the brewer's spent grain extract and having antibacterial, skin-whitening, antioxidant, and melanogenesis inhibitory effects by adding the processed brewer's spent grain product as an active ingredient.

Additionally, the processed brewer's spent grain product obtained by the method described above may be included as an active ingredient to prepare a cosmetic composition.

### Advantageous Effects

According to an embodiment of the present disclosure, a cosmetic ingredient having a skin-whitening efficacy and a cosmetic composition containing the same can be processed so that fiber and active ingredients abundantly contained in brewer's spent grain can be used to the fullest extent in the cosmetics industry.

Accordingly, the brewer's spent grain, according to the embodiment of the present disclosure, can be used as a cosmetic composition in addition to existing uses, so 1000 of the brewer's spent grain can be used without generating additional waste, and an economical and environmentally friendly process of preparing skin-whitening cosmetics thus can be arranged.

### Description of Drawings

FIG. 1 is a flowchart for explaining a processed brewer's spent grain product, a cosmetic composition using the same, and a preparation method thereof, according to an embodiment of the present disclosure;
FIGS. 2 and 3 are exemplary diagrams of a processed brewer's spent grain product and a cosmetic composition using the same, according to an embodiment of the present disclosure;
FIG. 4 is a diagram for explaining an encapsulation process of a processed brewer's spent grain product according to an embodiment of the present disclosure, and FIG. 5 is an exemplary diagram of the processed brewer's spent grain product obtained through the encapsulation process;
FIG. 6 is a flowchart for explaining a preparation method of a cosmetic composition containing a scrub and a capsule of a processed brewer's spent grain product, according to an embodiment of the present disclosure, in more detail;
FIG. 7 is a flowchart for explaining a preparation method of a cosmetic composition containing an extract of a processed brewer's spent grain product, according to an embodiment of the present disclosure, in more detail;
FIGS. 8 and 9 show diagrams for explaining the efficacy of a cosmetic containing a brewer's spent grain extract, according to an embodiment of the present disclosure;
FIG. 10 shows experimental data to compare the extraction yield depending on a dry processing method of brewer's spent grain, according to an embodiment of the present disclosure;
FIG. 11 shows experimental comparative data for efficacy with varying extraction concentrations of a brewer's spent grain extract, according to an embodiment of the present disclosure, during extraction for hot water extraction and varying processing concentrations of a processed product of the brewer's spent grain extract extracted through the hot water extraction; and
FIG. 12 shows analysis charts for the effect of a processed brewer's spent grain product using a brewer's spent grain extract, according to an embodiment of the present disclosure, on growth factors related to hair growth and hair growth promotion of human hair dermal papilla cells by processing concentration.

### Mode for Invention

Hereinafter, a processed brewer's spent grain product, a cosmetic composition using the same, and a preparation method thereof, according to an embodiment of the present disclosure, will be described in more detail with reference to the drawings.

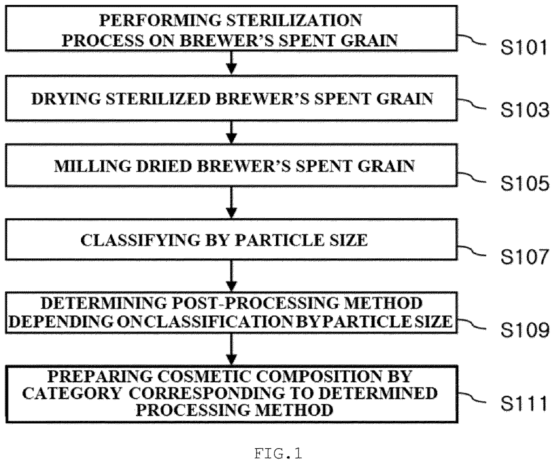

FIG. 1 is a flowchart for explaining a processed brewer's spent grain product, a cosmetic composition using the same, and a preparation method thereof, according to an embodiment of the present disclosure.

Brewer's spent grain contains all solids separated through filtration from wort, and the solids include residues of an adjunct and barley malt. When using corn grits as an adjunct, brewer's spent grain is composed mainly of: a pericarp and husk portion of barley; and a non-starch portion of corn. Brewer's spent grain is a lignocellulose-based material typically including lipids, lignin, proteins, cellulose, hemicellulose, and some ash. In the description and claims of the present disclosure, the term "brewer's spent grain (BSG)" will be used according to the above definition provided herein.

Additionally, barley is the main raw material used in beer production, and other grains, such as corn and rice, are typically used in combination with malted barley. During a brewing process, the starchy endosperm of such grains is enzymatically broken down, leading to the liberation of fermentable carbohydrates (maltose, maltotriose, and low percentages of glucose), non-fermentable carbohydrates (dextrins), proteins, polypeptides, and amino acids. The resulting medium (which will be fermented to beer by yeast action) is known as wort. An insoluble grain component (mainly composed of the grain husk) is brewer's spent grain (BSG). In traditional brewing using lauter tuns, the BSG component forms a layer through which the resulting mash is filtered to produce wort and thus plays an important role. Therefore, the initial milling of malt is required to allow the grain husk to remain intact such that a proper filter is formed. Although numerous small or craft breweries still use such a mash filtration method nowadays, many larger breweries use mash filters that depend less on the filtration function of the BSG, enabling the malt to be milled more extensively.

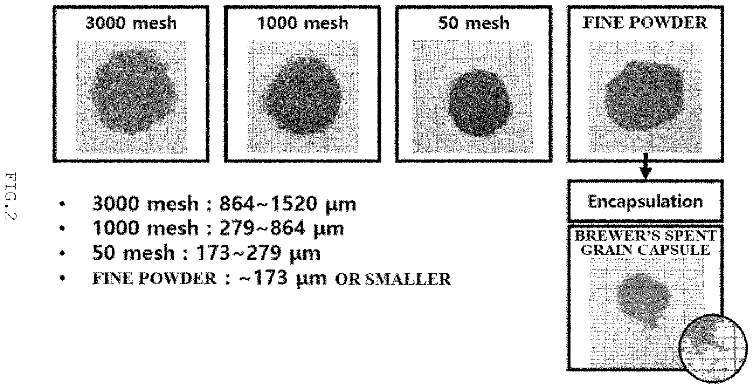

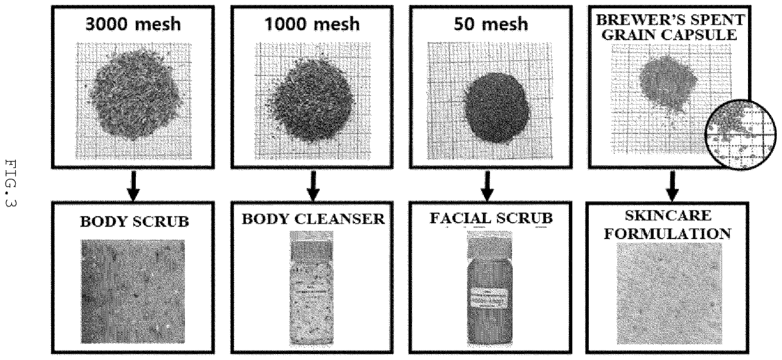

FIG. 1 is to explain a process of processing a cosmetic composition using such a brewer's spent grain, and FIGS. 2 and 3 are exemplary diagrams of a processed brewer's spent grain product and a cosmetic composition using the same, according to an embodiment of the present disclosure.

First, referring to FIG. 1, the brewer's spent grain is allowed to undergo a sterilization process (S101).

The sterilization is preferably performed in a sterilization environment at a temperature of 121 degrees for 0.5 to 1.0 hour during the sterilization process.

Then, a drying process is performed on the sterilized brewer's spent grain (S103).

Although the drying is preferably performed in a drying environment at a temperature in a range of 50 to 70 degrees for 2 to 6 hours during the drying process, various drying methods may be applied.

More specifically, the drying environment, according to Example 1, may involve, for example, a method of drying the brewer's spent grain obtained through the sterilization process at a temperature of 45 degrees for 24 hours.

Additionally, the drying environment, according to Example 2, may involve, for example, a method of performing freeze drying for 22 hours and then drying the resulting product obtained through the freeze drying at a temperature of 75 degrees for 2 hours.

Additionally, the drying environment, according to Example 3, may involve, for example, a method of performing hot air drying.

Next, a milling process is performed on the dried brewer's spent grain (S105).

In this case, the resulting brewer's spent grain obtained through the milling process may have a size corresponding to a mesh depending on various particle size conditions.

Accordingly, a classification process is performed on the milled brewer's spent grain by the milling particle size thereof (S107).

More specifically, referring to FIG. 2, the brewer's spent grain is preferably divided according to the following particle size conditions: fine powder, 50 mesh, 1000 mesh, 3000 mesh, and the like. For example, the fine powder condition corresponds to a case of the size being 173 micrometers or smaller, 50 mesh corresponds to a case of the size being in a range of 173 to 279 micrometers, 1000 mesh corresponds to a case of the size being in a range of 279 to 864 micrometers, and 3000 mesh corresponds to a case of the size being in a range of 864 to 1520 micrometers.

Then, a post-processing method is determined depending on the classified particle size of the brewer's spent grain (S109), and a cosmetic composition by each category corresponding to the determined processing method is prepared (S111).

More specifically, referring to FIG. 3, the mesh for each particle size condition may be used to prepare cosmetics for different categories by different processing methods.

For example, as shown in FIG. 3, the brewer's spent grain may be formulated into a body scrub in the case of the size being 3000 mesh, formulated into a body cleanser in the case of the size being 1000 mesh, and formulated into a facial scrub in the case of the size being 50 mesh. The fine powder may be processed into a brewer's spent grain capsule and formulated into skincare formulations mixed with various other cosmetic ingredients.

Additionally, as confirmed through various experimental results to be described later, the efficacy of the processed brewer's spent grain product to be processed may vary depending on the processing concentrations thereof. Accordingly, in the cosmetic composition according to the embodiment of the present disclosure, the processing concentration of the post-processed brewer's spent grain may be adjusted by the classified particle size, thereby maximizing the effect thereof depending on purposes including skin-whitening, anti-oxidation, hair growth, and hair growth promotion.

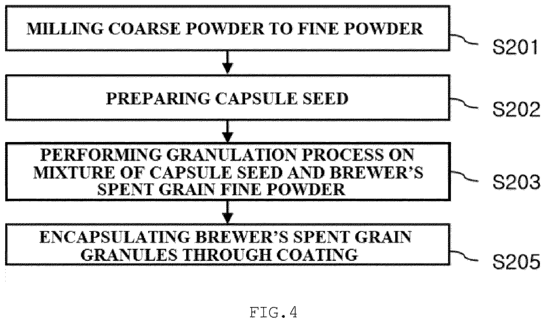

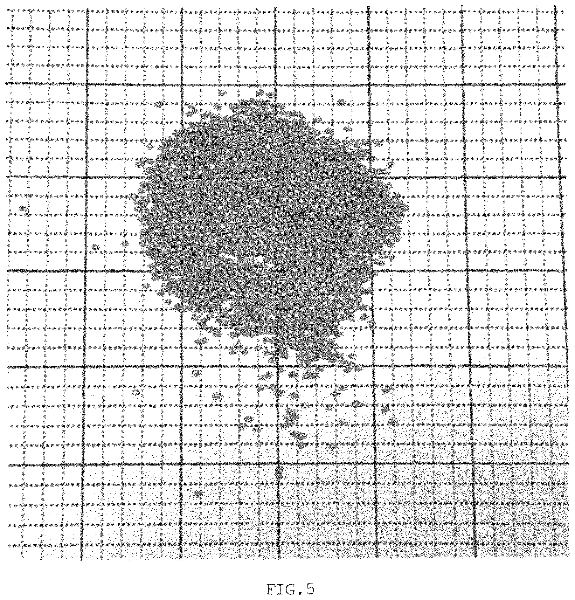

FIG. 4 is a diagram for explaining an encapsulation process of the processed brewer's spent grain product according to an embodiment of the present disclosure, and FIG. 5 is an exemplary diagram of the processed brewer's spent grain product obtained through the encapsulation process.

Referring to FIG. 4, the sterilization, drying, and milling processes described above in S101 and S103 are first performed on the brewer's spent grain. Then, when obtaining a coarse powder, a fine milling process is performed for encapsulation (S201).

Depending on the fine milling process, powder having a size of 173 micrometers or smaller, which is smaller than 50 mesh, may be obtainable.

Then, a capsule seed for an encapsulation process is prepared (S202). The capsule seed may contain a capsule polymer for preparing a cosmetic capable of containing the fine powder of the brewer's spent grain.

Next, the capsule seed is mixed with the fine powder of the brewer's spent grain, and then a granulation process is performed on the resulting mixture (S203).

Next, the resulting brewer's spent grain granule obtained through the granulation process is encapsulated through a coating process (S205).

FIG. 5 shows the encapsulated brewer's spent grain granule, which may be applied to skincare formulations of the cosmetic using the brewer's spent grain described above. Examples of the skincare formulations may include a lotion, a toner, an emulsion, a cream, a massage cream, an essence, a facial mask, and the like. With the addition of the encapsulated brewer's spent grain granule, skincare products using the processed brewer's spent grain product may be prepared.

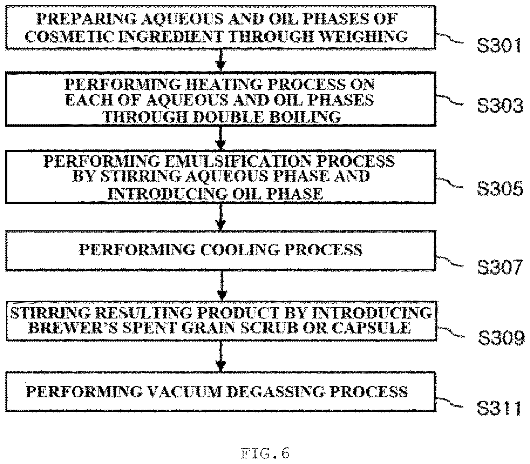

FIG. 6 is a flowchart for explaining a preparation method of a cosmetic composition containing a scrub and a capsule of the processed brewer's spent grain product, according to an embodiment of the present disclosure, in more detail.

Referring to FIG. 6, the aqueous and oil phases of a cosmetic ingredient for preparing the cosmetic containing the scrub and the capsule are separately weighed and prepared (S301).

Then, a heating process through double boiling is performed on each of the aqueous and oil phases (S303).

In this case, the heating process through double boiling is preferably performed at a temperature of about 90 degrees.

Then, as the aqueous phase is stirred and the oil phase is introduced, an emulsification process is performed (S305).

Next, a cooling process is performed on the resulting composition obtained through the emulsification process (S307).

In this case, the cooling process is preferably performed at a temperature of about 50 degrees or lower.

Then, the brewer's spent grain scrub or capsule pre-formulated in the same manner as illustrated in FIG. 1 or 4 is introduced into the resulting composition obtained through the cooling process to perform stirring (S309).

Next, a vacuum degassing process is performed on the resulting stirred composition (S311).

The cosmetic composition containing the scrub and capsule of the processed brewer's spent grain product, prepared by the preparation method of the cosmetic composition containing the scrub and capsule as described above, may be formulated into various products in the forms of a body scrub, a facial scrub, a cleanser, a scalp scaling product, a body wash, a facial wash, a shampoo, and the like.

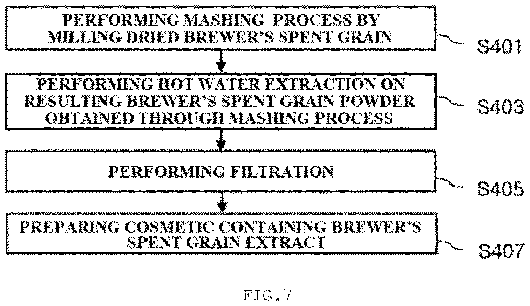

FIG. 7 is a flowchart for explaining a preparation method of a cosmetic composition containing an extract of the processed brewer's spent grain product, according to an embodiment of the present disclosure, in more detail.

Referring to FIG. 7, S101 to S103 steps illustrated in FIG. 1 are performed, followed by milling the resulting dried brewer's spent grain through a mashing process (S401).

In this case, the resulting brewer's spent grain obtained through the milling process may, for example, have a size corresponding to 50 mesh, indicating that the brewer's spent grain is milled to a size in a range of about 173 to 279 micrometers.

Next, hot water extraction is performed on the resulting brewer's spent grain powder obtained through the mashing process (S403).

More specifically, in hot water extraction, the brewer's spent grain obtained through the mashing process may be in a dried state by various methods. Thus, the hot water extraction is preferably performed by mixing an amount corresponding to a preset weight ratio, weight-to-volume ratio, or concentration ratio depending on each state.

Preferably, 200 mL of distilled water (DW) is mixed per 10 g of the resulting brewer's spent grain obtained through the mashing process to prepare a mixture for hot water extraction with a weight-to-volume ratio of 5%. With the extraction of the resulting mixture by an extraction method in an environment of a shaking water bath at a temperature of 70 degrees for 3 hours at 50 rpm, the efficacy and effect of a brewer's spent grain extract, which will be described later, may be maximized.

Next, filtration of the resulting brewer's spent grain extract obtained through the hot water extraction is performed (S405).

The filtration process may be performed on the resulting extracted solution of the brewer's spent grain extract extracted through the hot water extraction in the same manner described above, using filter paper made of quantitative filter paper. Preferably, the filter paper used may have a pore size of 20 µm.

Additionally, the brewer's spent grain extract on which the filtration is performed may be processed into a mixture capable of being stirred and mixed with other cosmetic composition formulations through freeze drying for 48 hours.

Accordingly, the filtered brewer's spent grain extract is stirred and mixed with other cosmetic composition formulations to prepare a cosmetic containing the brewer's spent grain extract (S407).

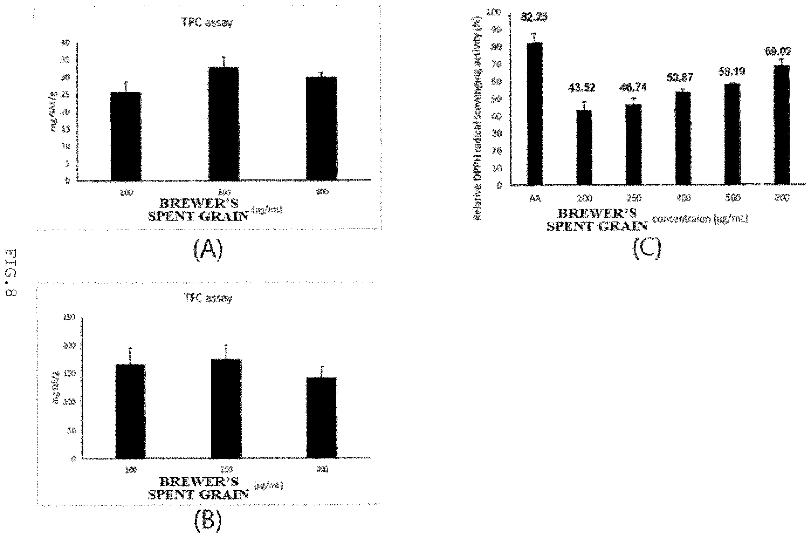

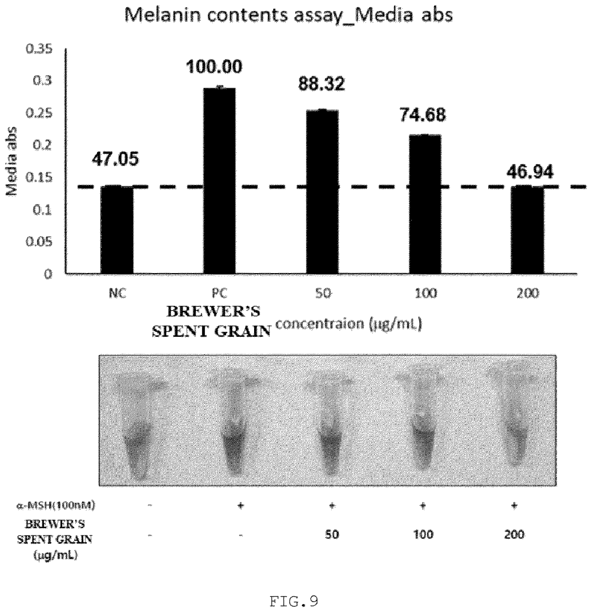

FIGS. 8 and 9 show diagrams for explaining the efficacy of the cosmetic containing the brewer's spent grain extract, according to the embodiment of the present disclosure.

First, FIG. 8A is to compare the total polyphenol content of the processed brewer's spent grain product prepared according to the embodiment of the present disclosure, confirming that this content is exhibited within a range of about 25 to 35 mg GAE/g with varying concentrations of the brewer's spent grain.

Additionally, FIG. 8B is to compare the total flavonoid content of the processed brewer's spent grain product prepared according to the embodiment of the present disclosure, confirming that this content is exhibited within a range of about 150 to 200 mg QE/g with varying concentrations of the brewer's spent grain.

Flavonoid and polyphenol components are known to have excellent antibacterial and skin-whitening effects, so the cosmetic containing the brewer's spent grain extract using the processed brewer's spent grain product, according to the embodiment of the present disclosure, is confirmed to have sufficient antibacterial and skin-whitening effects.

On the other hand, FIG. 8C is to compare the antioxidant activity of the processed brewer's spent grain product, prepared according to the embodiment of the present disclosure, by the DPPH assay test, confirming that the antioxidant activity gradually increases depending on the concentration of the brewer's spent grain. Therefore, the cosmetic containing the brewer's spent grain extract using the processed brewer's spent grain product, according to the embodiment of the present disclosure, is confirmed to have sufficient antioxidant effect depending on the concentration thereof.

Additionally, FIG. 9 shows the results of testing the melanogenesis inhibitory efficacy of the processed brewer's spent grain product prepared according to the embodiment of the present disclosure.

As shown in FIG. 9, melanin increased by α-MSH in B16F10 cells, which are melanin-producing cells, is confirmed to be reduced in a concentration-dependent manner by a processed product of the brewer's spent grain extract extracted through the hot water extraction illustrated below.

In particular, even in the case where the processed brewer's spent grain extract is processed at a low processing concentration of 200 µg/mL, melanogenesis is confirmed to be inhibited to a level equivalent to that in the case of a negative control (NC).

Accordingly, the cosmetic containing the brewer's spent grain extract using the processed brewer's spent grain product, according to the embodiment of the present disclosure, is confirmed to have an excellent melanogenesis inhibitory effect.

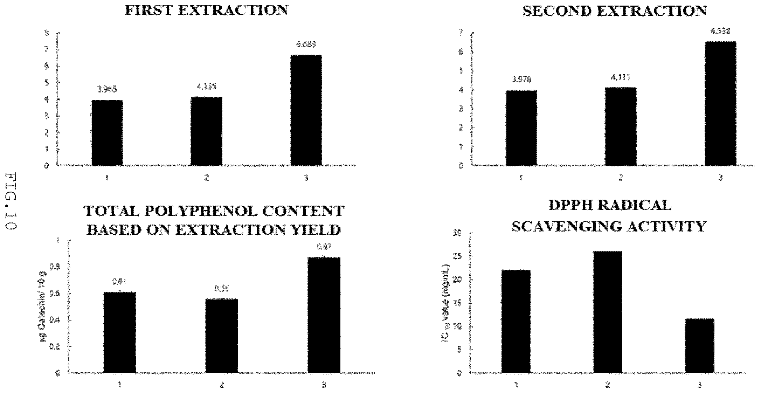

On the other hand, FIG. 10 shows experimental data to compare the extraction yield depending on a dry processing method of the brewer's spent grain, according to the embodiment of the present disclosure.

As described above, the environment of the dry processing method may differ in the case of the brewer's spent grain. Thus, in this experimental data, the extraction yield and effect according to each case of Examples 1, 2, and 3 are measured and shown.

More specifically, the drying environment, according to Example 1, involved a method of drying the resulting brewer's spent grain obtained through the sterilization process at a temperature of 45 degrees for 24 hours, and the drying environment, according to Example 2, involved a method of performing freeze drying for 22 hours and then drying the resulting product obtained through the freeze drying at a temperature of 75 degrees for 2 hours. Additionally, the drying environment, according to Example 3, involved a method of performing hot air drying.

Then, 200 mL of distilled water (DW) was mixed per 10 g of the resulting brewer's spent grain obtained through the drying and mashing processes depending on each of the three drying methods to prepare a mixture for hot water extraction with a weight-to-volume ratio of 5%. The resulting mixture was extracted by an extraction method in an environment of a shaking water bath at a temperature of 70 degrees for 3 hours at 50 rpm. Then, a filtration process was performed on the extracted solution of the brewer's spent grain extract extracted through the hot water extraction, using filter paper made of quantitative filter paper. In this case, the filter paper used has a pore size of 20 µm.

As shown in FIG. 10, the yield of the brewer's spent grain extract depending on the drying environment was confirmed to be significantly higher in the case of Example 3 involving hot air drying. Accordingly, the polyphenol content was confirmed to increase similarly. However, the antioxidant activity based on the DPPH radical scavenging activity was confirmed to be relatively high in the case of Examples 1 and 2.

Accordingly, the preparation method of the brewer's spent grain extract, according to the embodiment of the present disclosure, may diversify the preparation process by selecting the drying environment based on the drying method during the preparation step from one of the following methods: performing drying at a first temperature of 40 degrees or higher for a predetermined period as in Example 1; performing freeze drying for a first period and then drying the resulting product obtained through the freeze drying at a second temperature, which is a high temperature of 70 degrees or higher, for a second period, shorter than the first period, as in Example 2; and performing hot air drying for a predetermined period as in Example 3. By adjusting the selective drying method accordingly, the target polyphenol content and target antioxidant activity may be appropriately controlled according to the characteristics of the cosmetic composition. Therefore, it is seen that the yield of the brewer's spent grain extract based on the target efficacy may be optimized by diversifying the drying method.

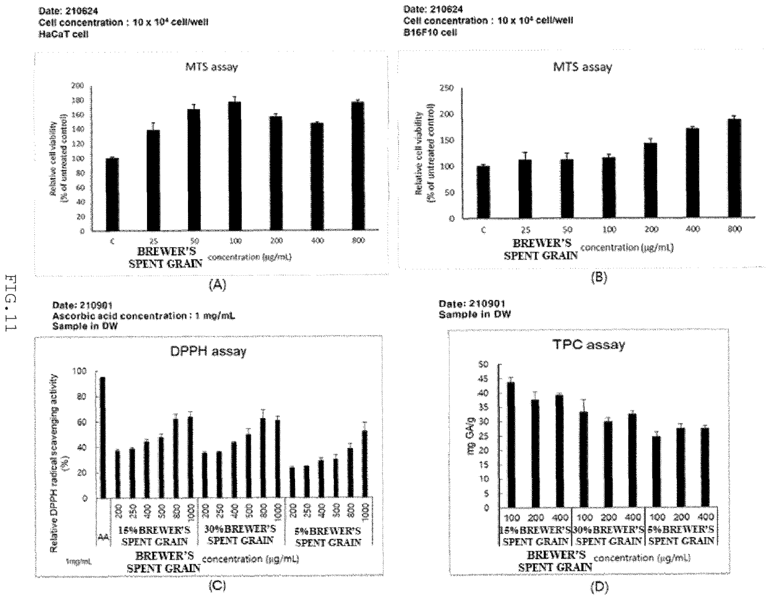

On the other hand, FIG. 11 shows experimental comparative data for efficacy with varying extraction concentrations of the brewer's spent grain extract, according to the embodiment of the present disclosure, during extraction for hot water extraction and varying processing concentrations of the processed product of the brewer's spent grain extract extracted through the hot water extraction.

Referring to FIG. 11, the extraction concentrations of the brewer's spent grain extract were set to 5%, 15%, and 30% for the experiment, and the weight-to-volume ratios of the processing concentration by extraction concentration were set to 25, 50, 100, 200, 400, and 800 ug/mL for the experiment. Distilled water (DW) was used as a solvent. Assuming that the specific gravity of typical distilled water is 1, the respective processing concentrations may be expressed by concentrations of 25, 50, 100, 200, 400, and 800 parts per million (ppm) based on the total weight of the composition.

First, FIGS. 11A and 11B are to test cell safety, showing MTT assay charts based on cell viability analysis performed on human adult low calcium high temperature (HaCaT) cells for measuring the impact of each processing concentration on the human body and B16F10 cells for measuring melanin, when the extraction concentration of the brewer's spent grain extract extracted through the hot water extraction is 5%.

As shown in FIGS. 11A and 11B, the relative cell viability of the HaCaT cells was shown to be the highest in the case of the concentration being 100 ppm, that is, 100 µg/mL. Additionally, in the case of the B16F10 cells, it was confirmed that the higher the concentration, the higher the cell viability in a concentration-dependent manner.

Additionally, FIG. 11C shows the measurement results of DPPH radical scavenging activity at each processing concentration for the cases where the hot water extraction concentrations of the brewer's spent grain extract extracted through hot water extraction are 5%, 15%, and 30%, and FIG. 11D shows the measurement results of the polyphenol content measured for the same cases. The ascorbic acid concentration in a standard control solution in FIG. 11C is 1 mg/mL. In the experiment shown in FIG. 11D, gallic acid was used as a standard control solution.

On the other hand, referring to FIGS. 11C and 11D, in the case of the processing concentration for each extraction concentration being 100 µg/mL, that is, when being processed at a concentration of 100 ppm, the polyphenol content was the highest, and the DPPH radical scavenging activity was high. Additionally, when classified by the extraction concentrations during the hot water extraction, it was confirmed that in the case of the extraction concentration being 15%, the polyphenol content was the highest, and the DPPH radical scavenging activity was high. These values decreased in the order of the cases where the extraction concentrations were 30% and 5%.

Therefore, depending on the target efficacy and effect, when maximizing the polyphenol content and the inhibitory effect of melanin secretion, it was confirmed to be the most preferable when setting the extraction concentration of the brewer's spent grain, according to the embodiment of the present disclosure, during the hot water extraction to 15% and mixing the composition at a processing concentration of 100 µg/mL.

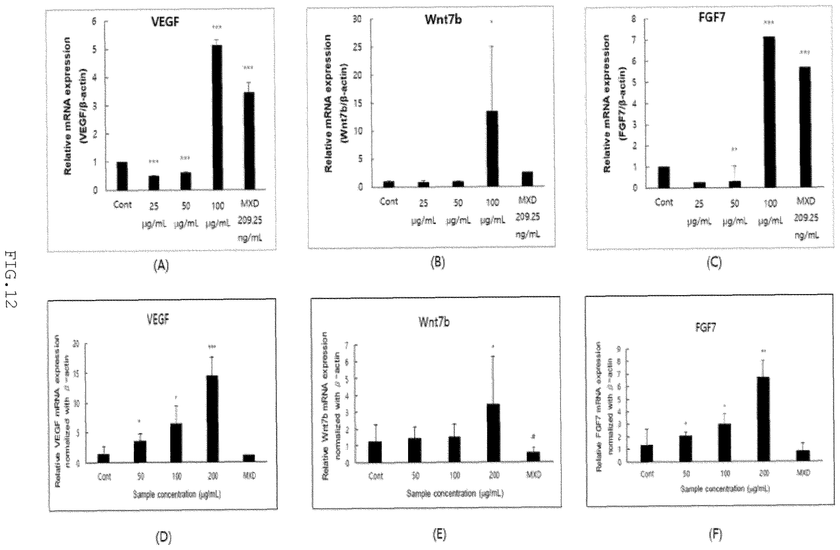

On the other hand, FIG. 12 shows analysis charts for the effect of the processed brewer's spent grain product using the brewer's spent grain extract, according to the embodiment of the present disclosure, on growth factors related to hair growth and hair growth promotion of human hair dermal papilla cells by processing concentration.

Referring to FIG. 12, the cells used in this experiment were human hair dermal papilla cells, and the mRNA expression levels of growth factors by processing concentration of the processed brewer's spent grain product using each brewer's spent grain extract were measured using a qPCT method. Three markers were used as biomarkers: VEGF (involved in cell proliferation of human hair dermal papilla cells), Wnt7b (involved in hair growth cycling and homeostasis of hair follicle stem cells), and FGF7 (involved in cell proliferation of human hair dermal papilla cells). For this efficacy analysis method, the 2018 paper Effect of *Cinnamomum osmophloeum* Kanehira Leaf Aqueous Extract on Dermal Papilla Cell Proliferation and Hair Growth (Cell Transplant. 2018 Feb;27(2):256-263, Tung-Chou Wen, et al.) may be referred.

First, FIGS. 12A, 12B, and 12C show the measurement results obtained by reacting the processed brewer's spent grain product extracted through the hot water extraction according to an extraction concentration of 15% in the human hair dermal papilla cells (HDPC) at a cell concentration of 2 × 10^5 cells/mL. In this case, 24 hours of starvation and 24 hours of prep time were required. The biomarker levels identified by each processing concentration are shown. Additionally, for a positive control, the comparative results based on minoxidil (MXD) at a concentration of 209.25 ng/mL (1 µM), which is known to be the most effective on dermal papilla cells (DPC), were used.

Referring to FIG. 12A, the expression level of VEGF is confirmed to increase significantly at a processing concentration of 100 ug/mL compared to other concentrations. In this case, it is confirmed that the effect is 5 times greater than that in the case of the control, and there is a significant increase in the effect by about 1.5 times greater than that in the case of the positive control.

Referring to FIG. 12B, the expression level of Wnt7b is also confirmed to increase significantly at a processing concentration of 100 ug/mL compared to other concentrations. In this case, it is confirmed that the effect is 13.4 times greater than that in the case of the control, and there is a significant increase in the effect by about 5.4 times greater than that in the case of the positive control.

Additionally, referring to FIG. 12C, the expression level of FGF7 is also confirmed to increase significantly at a processing concentration of 100 ug/mL compared to other concentrations. In this case, it is confirmed that the effect is 7 times greater than that in the case of the control, and there is a significant increase in the effect by about 1.2 times greater than that in the case of the positive control.

Additionally, FIGS. 12D, 12E, and 12F show the measurement results obtained by reacting the processed brewer's spent grain product extracted through the hot water extraction according to an extraction concentration of 15% in the human hair dermal papilla cells (HDPC) at a cell concentration of 3 × 10^5 cells/dish on different days. In this case, 24 hours of starvation was required. The biomarker levels identified by each processing concentration are shown. Similarly, for a positive control, the comparative results based on minoxidil (MXD) at a concentration of 209.25 ng/mL (1 µM), which is known to be the most effective on dermal papilla cells (DPC), were used.

Referring to FIG. 12D, the expression level of VEGF is confirmed to increase significantly at a processing concentration of 200 ug/mL compared to other concentrations. In this case, it is confirmed that the effect is about 11 times greater than that in the case of the control, and there is a significantly large difference compared to that in the case of the positive control.

Referring to FIG. 12E, the expression level of Wnt7b is also confirmed to increase significantly at a processing concentration of 200 ug/mL compared to other concentrations. In this case, it is confirmed that the effect is 5 times greater than that in the case of the control, and there is also a significantly large difference compared to that in the case of the positive control.

Additionally, referring to FIG. 12F, the expression level of FGF7 is also confirmed to increase significantly at a processing concentration of 200 ug/mL compared to other concentrations. In this case, it is confirmed that the effect is 3 times greater than that in the case of the control, and there is also a significantly large difference compared to that in the case of the positive control.

Referring to these experimental results, in the case where the processed brewer's spent grain product is extracted through the hot water extraction at an extraction concentration of 15%, the efficacies related to hair growth and hair growth promotion on human hair dermal papilla cells are confirmed to be expressed at the highest level within the processing concentration in a range of 100 to 200 µg/mL.

Although specific embodiments of the present disclosure have been described hereinabove, various modifications are possible without departing from the scope of the present disclosure. Therefore, the scope of the present disclosure should not be limited to the embodiments described above, but should be determined by the scope of the appended claims as well as equivalents thereof.

As described above, although the present disclosure has been described with reference to limited embodiments and drawings, the present disclosure is not limited to the above embodiments, and those skilled in the art may appreciate that various alternatives and modifications are possible from these descriptions. Accordingly, the spirit of the present disclosure should be understood only by the scope of the appended claims, and all equivalents, additions, or substitutions thereof fall within the scope of the spirit of the present disclosure.

## Claims

1. A method of preparing a processed brewer's spent grain product to prepare a cosmetic composition, the method comprising:
performing a sterilization process on a brewer's spent grain raw material;
selectively performing a drying process on the sterilized brewer's spent grain raw material depending on a predefined drying environment;
performing a milling process on the resulting brewer's spent grain raw material obtained through the drying process;
classifying the milled brewer's spent grain raw material by a particle size condition;
determining a post-processing method corresponding to the classified particle size condition; and
preparing a processed brewer's spent grain product for preparing a cosmetic composition by category, depending on the determined post-processing method.

2. The method of claim 1, wherein the particle size condition corresponds to a milling size condition, and
the milling size condition comprises at least one among the following conditions: less than 50 mesh, 50 mesh, 1000 mesh, and 3000 mesh.

3. The method of claim 2, wherein the category of the cosmetic composition prepared varies with the milling size condition.

4. The method of claim 2, wherein when the milling size condition corresponds to a fine powder, the post-processing method comprises: a fine milling process performed on the milled brewer's spent grain raw material; a granulation process performed on a mixture of a capsule seed and the resulting fine powder of the brewer's spent grain raw material; and an encapsulation process performed on the resulting product through coating, and
the brewer's spent grain raw material obtained through the encapsulation process is used as a skincare formulation of the cosmetic composition.

5. The method of claim 2, wherein when the milling size condition corresponds to 3000 mesh, the category of the cosmetic composition corresponds to a body scrub,
when the milling size condition corresponds to 1000 mesh, the category of the cosmetic composition corresponds to a body cleanser, and
when the milling size condition corresponds to 50 mesh, the category of the cosmetic composition corresponds to a facial scrub.

6. The method of claim 1, wherein the post-processing method comprises a process of obtaining a brewer's spent grain extract from the milled brewer's spent grain raw material by performing hot water extraction and filtration.

7. The method of claim 6, wherein the obtaining process comprises a process of preparing a mixture for the hot water extraction in which distilled water and the milled brewer's spent grain raw material at an extraction concentration in a range of 15% to 30% are mixed, and then performing the hot water extraction on the resulting mixture, thereby obtaining the brewer's spent grain extract.

8. The method of claim 7, wherein the cosmetic composition is formulated into a cosmetic containing the brewer's spent grain extract and having a hair growth or hair growth promotion effect on human hair dermal papilla cells by:
mixing distilled water and the milled brewer's spent grain raw material at an extraction concentration of 15% to prepare the mixture for the hot water extraction; and
adding the processed brewer's spent grain product in which the brewer's spent grain extract extracted from the resulting mixture through the hot water extraction is mixed and processed with distilled water according to a processing concentration of 100 ppm or 200 ppm, as an active ingredient.

9. The method of claim 7, wherein the cosmetic composition is formulated into a cosmetic containing the brewer's spent grain extract and having antibacterial, skin-whitening, antioxidant, and melanogenesis inhibitory effects by adding the processed brewer's spent grain product in which the brewer's spent grain extract is mixed and processed with distilled water according to a processing concentration of 100 ppm, as an active ingredient.

10. A cosmetic composition comprising a processed brewer's spent grain product obtained by the method of claim 1 as an active ingredient.

11. A method of preparing a processed brewer's spent grain product to prepare a cosmetic composition, the method comprising:
performing a sterilization process on a brewer's spent grain raw material;
performing a milling process by drying the sterilized brewer's spent grain raw material to form a brewer's spent grain powder;
obtaining a brewer's spent grain fine powder by milling the resulting milled brewer's spent grain powder to a preset or smaller size;
preparing a capsule seed comprising a capsule polymer for preparing a cosmetic capable of containing the brewer's spent grain fine powder;
obtaining a brewer's spent grain granule by mixing the capsule seed and the brewer's spent grain fine powder;
performing an encapsulating process on the brewer's spent grain granule through a coating process; and
obtaining a processed brewer's spent grain product to use the resulting brewer's spent grain raw material obtained through the encapsulation process as a skincare formulation of a cosmetic composition.

12. The method of claim 11, wherein the skincare formulation of the cosmetic composition is used as a skincare formulation of a skincare product comprising at least one among a lotion, a toner, an emulsion, a cream, a massage cream, an essence, and a mask pack.

13. The method of claim 11, wherein the performing of the milling process comprises selectively formulating a drying environment depending on a drying method, and
the drying environment is any one selected from among the following environments: performing drying at a first temperature of 40 degrees or higher for a predetermined period; performing freeze drying for a first period and then drying the resulting product obtained through the freeze drying at a second temperature of 70 degrees or higher for a second period, shorter than the first period; and performing hot air drying for a predetermined period.

14. The method of claim 13, wherein the drying environment determined varies with a target polyphenol content and a target antioxidant activity based on a characteristic of the cosmetic composition.

15. A method of preparing a cosmetic composition containing a brewer's spent grain extract, the method comprising:
performing a sterilization process to dry a brewer's spent grain raw material and then performing a milling process on the resulting brewer's spent grain raw material;
pre-formulating a brewer's spent grain scrub or capsule by classifying the milled brewer's spent grain raw material by a particle size condition and performing a post-process depending on a post-processing method;
preparing an aqueous phase and an oil phase of a cosmetic ingredient for preparing a cosmetic containing a cosmetic composition containing the brewer's spent grain scrub or capsule by separately weighing each of the aqueous and oil phases;
performing a heating process on each of the aqueous and oil phases through double boiling and then performing an emulsification process by stirring the aqueous phase and introducing the oil phase;
performing a cooling process on the resulting composition obtained through the emulsification process;
performing stirring by introducing the pre-formulated brewer's spent grain scrub or capsule; and
performing a vacuum degassing process on the resulting stirred composition.

16. The method of claim 15, wherein the particle size condition corresponds to a milling size condition, and
the milling size condition comprises at least one among the following conditions: less than 50 mesh, 50 mesh, 1000 mesh, and 3000 mesh.

17. The method of claim 16, wherein a category of the cosmetic composition prepared varies with the milling size condition, and
the category comprises any one among a body scrub, a facial scrub, a cleanser, a scalp scaling product, a body wash, a facial wash, and a shampoo.
